Europäisches Patentamt

European Patent Office

Office européen des brevets

(19)

(11) Veröffentlichungsnummer: **0 224 844 B1**

# EUROPÄISCHE PATENTSCHRIFT

(12)

(45) Veröffentlichungstag der Patentschrift: **18.03.92**

(51) Int. Cl.$^5$: **C07K 1/06**, C07K 1/10, C07K 7/00

(21) Anmeldenummer: **86116325.1**

(22) Anmeldetag: **25.11.86**

(54) **Verfahren zur Herstellung von Peptiden unter der Verwendung von Perchloraten.**

(30) Priorität: **30.11.85 DE 3542442**

(43) Veröffentlichungstag der Anmeldung:
**10.06.87 Patentblatt 87/24**

(45) Bekanntmachung des Hinweises auf die
Patenterteilung:
**18.03.92 Patentblatt 92/12**

(84) Benannte Vertragsstaaten:
**AT BE CH DE ES FR GB GR IT LI LU NL SE**

(56) Entgegenhaltungen:

**CHEMISCHE BERICHTE, Band 104, Nr. 8, 14.
August 1971, Seiten 2445-2453, Verlag Chemie GmbH, Weinheim, DE; E. WÜNSCH et al.:
"Zur synthese des Sekretins, II, Darstellung
der Sequenz 12-27"**

(73) Patentinhaber: **HOECHST AKTIENGESELL-
SCHAFT
Postfach 80 03 20
W-6230 Frankfurt am Main 80(DE)**

(72) Erfinder: **König, Wolfgang, Dr.
Eppsteiner Strasse 25
W-6238 Hofheim am Taunus(DE)**
Erfinder: **Teetz, Volker, Dr.
An der Tann 20
W-6238 Hofheim am Taunus(DE)**

EP 0 224 844 B1

Rank Xerox (UK) Business Services

**Beschreibung**

Die Erfindung betrifft ein Verfahren zur Herstellung von geschützten argininhaltigen Peptiden durch Fragmentkupplung, das dadurch gekennzeichnet ist, daß man bei der jeweiligen Fragmentkupplung mindestens ein argininhaltigen Fragment als Perchlorat umsetzt.

Argininhaltige Fragmente im Sinne der vorliegenden Erfindung können sein:

1. Arginin, dessen COOH oder dessen $\alpha$-NH$_2$-Gruppe gegebenenfalls durch eine in der Peptidchemie üblichen Schutzgrupppe geschützt ist, oder

2. Arginin und weitere Aminosäuren enthaltende Segmente, deren funktionelle(n) Gruppe(n) gegebenenfalls durch (eine ) in der Peptidchemie übliche Schutzgruppe(n) geschützt ist (sind). Geeignete Schutzgruppen sind beispielsweise beschrieben in Schröder, Lübke, The Peptides, Band I, Acedemic Press, New York 1965, Seiten 3 - 75 und 137 - 270.

Die Fragmentkupplung erfolgt nach Standardverfahren, wie sie beispielsweise in Perspectives in Peptide Chemistry, Herausgeber Eberle et al., Karger, Basel 1981, Seiten 15 - 155 beschrieben sind. Bevorzugt ist die Kupplung in Gegenwart von Carbodiimiden, wie N, N'-Dicyclohexylcarbodiimid; N, N'-Di-tert.-butylcarbodiimid; N, N'-Diisopropylcarbodiimid; N-Ethyl-N'-(3-dimethylaminopropyl)carbodiimid oder N, N'-Bis(4-nitrophenyl)carbodiimid gegebenenfalls unter Zusatz von N-Hydroxysuccinimid, 1-Hydroxybenzotriazol, 3-Hydroxy-4-oxo-3,4-dihydro-1,2,3-benzotriazin oder anderer racemisierungssenkender Verbindungen. Die Kupplung kann sowohl "klassisch" in Lösung als auch nach der Festkörpermethode (Merrifield) mit einem während des Syntheseablaufs kovalent an ein Harz gebundenem Fragment erfolgen.

Die Verwendung höherer, an den Seitenketten geschützter Peptide als Zwischenprodukte in der Peptidsynthese ist häufig durch deren schlechte Löslichkeit erschwert. Oft sind diese Peptide so unlöslich, daß an eine Weitersynthese nicht mehr zu denken ist. Die schlechte Löslichkeit erhöht die Reaktionsdauer und verringert die Ausbeute. Diese Schwierigkeiten treten beispielsweise bei der Synthese von Sekretin auf.

Sekretin, ein Hormon aus dem Duodenum, ist ein Heptacosapeptid der Formel

H-His-Ser-Asp-Gly-Thr-Phe-Thr-Ser-Glu-Leu-Ser-Arg-Leu-Arg-
Asp-Ser-Ala-Arg-Leu-Gln-Arg-Leu-Leu-Gln-Gly-Leu-Val-NH$_2$

(Eur. J. Biochem. 15, 1970, Seite 513 - 519). Sekretin stimuliert die Bicarbonatproduktion der Bauchspeicheldrüse und hemmt die gastrinstimulierende Magensäuresekretion.

Sekretin wurde bereits stufenweise unter Verwendung der p-Nitrophenylester-Methode (J.Am. Chem. Soc, 89, 1967, Seite 6753 - 6757) und der "Repetitive Excess Mixed Anhydride" (REMA) Methode (Helv. Chim. Acta 59, 1976, Seite 1112 - 1126) synthetisiert. Die Verwendung von Segmenten zur Synthese von Sekretin bedarf möglichst racemisierungsfreier Kupplungsmethoden. So konnte bereits mit Hilfe der Azid-Methode (J. Am. Chem. Soc. 90, 1968, Seite 4711 - 4715) und der Dicyclohexylcarbodiimid/N-Hydroxysuccinimid(DCC/HONSu)Methode (Chem. Ber. 105, 1972, Seite 2508 - 2514) Sekretin aufgebaut werden. Weitere Varianten der DCC-Kupplung bestanden in der Verwendung der racemisierungssenkenden und löslichkeitsvermittelnden Zusätze von 1-Hydroxybenzotriazol (HOBt) und 3-Hydroxy-4-oxo-3,4-dihydro-1,2,3-benztriazin (HOObt) (Chem. Ber. 107, 1974, Seite 215 - 231; Gut Hormones, ed. S. R. Bloom, 1978, Seite 165 - 168). Eine Sekretinsynthese an fester Phase wird in Int. J. Peptide Protein Res. 9, 1977, Seiten 63 - 70, beschrieben.

Die größte Schwierigkeit bei den oben genannten Segmentkupplungen verursacht die Schwerlöslichkeit der Fragmente, die wegen der hohen Verdünnung der Reaktanten lange Reaktionszeiten und Überschüsse des N-terminalen Fragments erforderlich machen. Durch Voraktivierung der N-terminalen Fragmente mit DCC/HOObt kann zwar die Reaktionszeit und meist auch das Reaktionsvolumen etwa verkleinert werden (Gut Hormones, ed. S. R. Bloom, 1978, Seite 165 - 168). Große Schwierigkeiten bereitet aber daneben auch die Abspaltung der für den intermediären Aminoschutz eingesetzten Benzyloxycarbonylgruppe (Z) durch katalytische Hydrierung. Auch hier ist die Schwerlöslichkeit die Hauptursache. Da Essigsäure als Lösungsmittel, wenn möglich, vermieden werden sollte (Essigsäure ist aus basischen Peptiden nur schwer zu entfernen und verursacht bei der Fragmentkupplung Acetylierung der Aminogruppen), mußte bereits auf kostspielige Lösungsmittel, wie z.B. Trifluorethanol ausgewichen werden (Gut Hormones, ed. S. R. Bloom, 1978, Seite 167).

Überraschenderweise wird die Synthese geschützter argininhaltiger Peptide durch Kupplung der oft schwerlöslichen Fragmente wesentlich erleichtert, wenn bei der Kupplung gemäß dem erfindungsgemäßen Verfahren wenigstens eine Peptidkomponente als Perchlorat eingesetzt wird. Perchlorate basischer Peptide besitzen eine ausgezeichnete Löslichkeit in polaren Lösungsmitteln, wie z.B. Dimethylformamid oder Dimethylacetamid, was für weitere Umsetzungen sehr vorteilhaft ist und die Raumausbeute verbessert. Ein geeignetes Reagens für die Einführung des Perchlorats sind neben der Perchlorsäure Perchlorate geeigneter Amine, vorzugsweise Pyridiniumperchlorat, das im Gegensatz zur wasserhaltigen Perchlorsäure wasserfrei eingewogen werden kann (Ber. dtsch. chem. Ges. 59, Seiten 448 - 455 (1926)).

Das Perchloration bindet sich an die stark basische Guanidinogruppe und das freiwerdende Pyridin geht in das Lösungsmittel. So kann durch Zusatz von Pyridiniumperchlorat die Löslichkeit argininhaltiger Peptide stark erhöht werden und dadurch zur Reaktion gebracht werden. Z.B. kann das äußerst schwer lösliche Z-Thr(Bu$^t$)-Ser(Bu$^t$)-Glu(OBu$^t$)-Leu-Ser(Bu$^t$)-Arg-Leu-OH unter Zusatz von Pyridiniumperchlorat mit DCC und HOObt in Dimethylacetamid voraktiviert werden. Auch das in organischen Lösungsmitteln praktisch unlösliche Arginin kann unter Zusatz von Pyridiniumperchlorat acyliert werden. Mit Argininhydrochlorid war dies nicht möglich.

Die Perchlorat-Ionen eignen sich nicht nur zur Erhöhung der Löslichkeit, sondern schützen die Guanidinogruppe auch vor Acylierung. So hat sich Pyridiniumperchlorat als sehr gutes Additiv für die Protonierung der Guanidinogruppe bei der Festphasensynthese unter Verwendung von Fmoc-Aminosäuren erwiesen, während Salzsäure bekanntlich für diesen Zweck nicht geeignet war (siehe E. Atherton, R.C. Sheppard und D. Wade, J. Chem. Soc., Chem. Commun 1983, 1060 - 1062).

Bevorzugt ist ein Verfahren zur Herstellung eines geschützten Sekretinderivats der Formel I,

$$
\begin{aligned}
&\text{R-His(R}^1\text{)-Ser(Bu}^t\text{)-Asp(OBu}^t\text{)-Gly-Thr(Bu}^t\text{)-Phe-Thr(Bu}^t\text{)-}\\
&\text{Ser(Bu}^t\text{)-Glu(OBu}^t\text{)-Leu-Ser(Bu}^t\text{)-Arg(HClO}_4\text{)-Leu-Arg(HClO}_4\text{)-}\\
&\text{Asp(OBu}^t\text{)-Ser(Bu}^t\text{)-Ala-Arg(HClO}_4\text{)-Leu-Gln-Arg(HClO}_4\text{)-}\\
&\text{Leu-Leu-Gln-Gly-Leu-Val-NH}_2 \quad \text{(I),}
\end{aligned}
$$

in der

    a) R und R$^1$ den Boc-Rest oder

    b) R den Boc-Rest und R$^1$ Wasserstoff oder

    c) R und R$^1$ den Adamantyloxycarbonyl-Rest bedeuten,

das dadurch gekennzeichnet ist, daß man ein Peptid der allgemeinen Formel IIa,

H-X-Leu-Leu-Gln-Gly-Leu-Val-NH$_2$ (IIa),

in der X

$$
\begin{aligned}
&\text{-Arg(HClO}_4\text{)-,}\\
&\text{-Arg(HClO}_4\text{)-Leu-Gln-Arg(HClO}_4\text{)-,}\\
&\text{-Arg(HClO}_4\text{)-Asp(OBu}^t\text{)-Ser(Bu}^t\text{)-Ala-Arg(HClO}_4\text{)-Leu-Gln-Arg}\\
&\text{(HClO}_4\text{)}
\end{aligned}
$$

oder

$$
\begin{aligned}
&\text{-Thr(Bu}^t\text{)-Ser(Bu}^t\text{)-Glu(OBu}^t\text{)-Leu-Ser (Bu}^t\text{)-Arg(HClO}_4\text{)-}\\
&\text{Leu-Arg(HClO}_4\text{)-Asp(OBu}^t\text{)-Ser(Bu}^t\text{)-Ala-Arg(HClO}_4\text{)-Leu-}\\
&\text{Gln-Arg(HClO}_4\text{)-}
\end{aligned}
$$

bedeutet, unter Zusatz von 3-Hydroxy-4-oxo-3,4-dihydro-1,2,3-benzotriazin (HOObt) im "Eintopfverfahren" mit den entsprechenden Peptiden mit freier Carboxylgruppe in polaren Lösungsmitteln wie beispielsweise Dimethylacetamid mit Dicyclohexylcarbodiimid und notwendigen Mengen einer tertiären Base, wie z.B. N-Ethylmorpholin umsetzt und die so synthetisierten Peptide aus Wasser unter Zusatz entsprechender Mengen Perchlorsäure und gegebenenfalls eines Perchlorats wie NaClO$_4$ als Perchlorate ausfällt.

3

Die hydrogenolytische Abspaltung der $N^\eta$-Nitro-Schutzgruppe des Arginins in Perchlorsäure ist aus J. Amer. Chem. Soc. 101, 1979, Seiten 1569 - 1576 bekannt.

Besonders bevorzugt ist ein Verfahren, das dadurch gekennzeichnet ist, daß das obengenannte Peptid der Formel IIa durch Hydrierung benzyloxycarbonylhaltigen Fragmente der allgemeinen Formel II,

Z-X-Leu-Leu-Gln-Gly-Leu-Val-NH$_2$ (II),

in der X -Arg(Z$_2$)-, -Arg(Z$_2$)-Leu-Gln-Arg(HClO$_4$)-, -Arg(Z$_2$)-Asp(OBu$^t$)-Ser(Bu$^t$)-Ala-Arg(HClO$_4$)-Leu-Gln-Arg-(HClO$_4$)- oder -Thr(Bu$^t$)-Ser(Bu$^t$)-Glu(OBu$^t$)-Leu-Ser(Bu$^t$)-Arg(HClO$_4$)-Leu-Arg(HClO$_4$)-Asp(OBu$^t$)-Ser(Bu$^t$)-Ala-Arg(HClO$_4$)-Leu-Gln-Arg (HClO$_4$)- bedeutet, in Dimethylacetamid unter Zusatz eines Palladiumkatalysators, wobei durch Zugabe einer perchlorsäurehaltigen Lösung der pH-Wert zwischen 4 und 6 gehalten wird, erhalten und dieses in der oben beschriebenen Weise weiter umgesetzt wird. Auch ein Zusatz äquivalenter Mengen Pyridiniumperchlorat ist möglich, wenn ein Autotitrator nicht verwendet werden kann.

Bei der Hydrierung von

Z-Arg(Z$_2$)-Leu-Leu-Gln-Gly-Leu-Val-NH$_2$ (III)

wurde bisher Eisessig als Lösungsmittel verwendet (Chem. Ber. 104, 1971, Seite 2441). Man verwendete etwa 350 ml Eisessig für 10 mmol der Substanz. Um die Essigsäure völlig zu entfernen, was notwendig ist, um eine Acetylierung in der nächsten Peptidbildungsstufe zu verhindern, muß man mit mindestens zwei Äquivalenten HBr oder HCl versetzen und anschließend mehrmals unter Zusatz von Pyridin aus Methanol und Essigester oder Diisopropylether umfällen. Zweckmäßiger wäre daher eine katalytische Hydrierung in Lösungsmitteln wie Methanol oder Dimethylacetamid unter Zusatz von HCl oder HBr. Versuche dieser Art scheiterten hierbei an der Schwerlöslichkeit von III in oben genannten Lösungsmitteln. Auch Suspensionen gingen während der Hydrierung nicht in Lösung. Überraschend war deshalb die Feststellung, daß das in Dimethylacetamid praktisch unlösliche III unter Zusatz von Perchlorsäure während der katalytischen Hydrierung unter Bildung von

H-Arg(HClO$_4$)-Leu-Leu-Gln-Gly-Leu-Val-NH$_2$ HClO$_4$ (IV)

schnell in Lösung geht. Für 10 mmol III sind nur 120 ml Dimethylacetamid nötig, also etwa ein Drittel der oben beschriebenen Essigsäuremenge. Neben der einfacheren Aufarbeitung hat das so gewonnene Peptid-diperchlorat IV den Vorteil der besseren Löslichkeit in polaren Lösungsmitteln, wie Dimethylacetamid und Dimethylformamid. Z.B. werden bei der Umsetzung von

Z-Arg(Z$_2$)-Leu-Gln-OH

mit dem entsprechenden Peptid-dihydrobromid (Chem. Ber. 104, 1971, Seite 2443 - 2444) für 10 mmol etwa 650 ml Dimethylformamid als Lösungsmittel verwendet, während ein 10 mmol Ansatz mit dem Peptid-diperchlorat IV nur 70 ml Dimethylacetamid, also ungefähr ein Zehntel des Lösungsmittels benötigt . Dadurch verkürzt sich die Reaktionszeit von 7 Tagen auf wenige Stunden und die Raumausbeute wird wesentlich erhöht.

Ähnliches beobachtet man auch bei den anderen Fragmenten. Bei der katalytischen Hydrierung von

Z-Arg(Z$_2$)-Leu-Gln-Arg(HClO$_4$)-Leu-Leu-Gln-Gly-Leu-Val-NH$_2$ (V)

zu

```
H-Arg(HClO4)-Leu-Gln-Arg(HClO4)-Leu-Leu-Gln-Gly-Leu-Val-
NH2  HClO4   (VI)
```

arbeitet man wie bei III. Für 10 mmol braucht man etwa 220 ml Dimethylacetamid, während das entsprechende Hydrobromid (Cheml. Ber. 104, 1971, Seite 2444) für 10 mmol 1540 ml Eisessig benötigt.

Bei der Kondensation von 10 mmol VI mit Z-Arg(Z$_2$)-Asp(OBu$^t$)-Ser(Bu$^t$)-Ala-OH werden nur noch 45 ml Dimethylacetamid als Lösungsmittel verwendet, während der etwa gleiche Ansatz mit dem entsprechenden Dekapeptid-trihydrochlorid noch 70 ml Lösungsmittel benötigt (Gut Hormones, ed. S.R. Bloom, 1978, Seite 166). Die katalytische Hydrierung des so erhaltenen Tetradekapeptids

```
Z-Arg(Z2)-Asp(OBut)-Ser(But)-Ala-Arg(HClO4)-Leu-Gln-Arg
(HClO4)-Leu-Leu-Gln-Gly-Leu-Val-NH2        (VII)
```

zu

$$\text{H-Arg(HClO}_4\text{)-Asp(OBu}^t\text{)-Ser(Bu}^t\text{)-Ala-Arg(HClO}_4\text{)-Leu-Gln-}$$
$$\text{Arg(HClO}_4\text{)-Leu-Leu-Gln-Gly-Leu-Val-NH}_2 \quad \text{HClO}_4 \qquad \text{(VIII)}$$

geht wiederum problemlos in Dimethylacetamid (10 mmol in 250 ml), während 10 mmol des entsprechenden Dihydrobromids in 2000 ml 80-proz. Essigsäure (Chem. Ber. 104, 1971, Seite 2450) und das Dihydrochlorid in dem kostspieligen Trifluorethanol (Gut Hormones, ed. S.R. Bloom, 1978, Seite 167) hydriert wurde.

Das schwer lösliche

Z-Thr(Bu$^t$)-Ser(Bu$^t$)-Glu(OBu$^t$)-Leu-Ser(Bu$^t$)-Arg-Leu-OH

löst sich leicht in Gegenwart von VIII, so daß auch hier die Kondensation zu

$$\text{Z-Thr(Bu}^t\text{)-Ser(Bu}^t\text{)-Glu(OBu}^t\text{)-Leu-Ser(Bu}^t\text{)-Arg(HClO}_4\text{)-}$$
$$\text{Leu-Arg(HClO}_4\text{)-Asp(OBu}^t\text{)-Ala-Arg(HClO}_4\text{)-Leu-Gln-}$$
$$\text{Arg(HClO}_4\text{)-Leu-Leu-Gln-Gly-Leu-Val-NH}_2 \qquad \text{(IX)}$$

im "Eintopf-Verfahren" in einem kleinen Lösungsmittelvolumen möglich ist. Ein 10 mmol Ansatz ist in 200 ml einer Dimethylformamid/Dimethylacetamid-Mischung gut durchführbar, während ein analoger Ansatz mit dem entsprechenden Tetrahydrobromid über 500 ml Lösungsmittel benötigte (Chem. Ber. 107, 1974, Seite 230).

Die katalytische Hydrierung von IX zu

$$\text{H-Thr(Bu}^t\text{)-Ser(Bu}^t\text{)-Glu(OBu}^t\text{)Leu-Ser-(Bu}^t\text{)-Arg(HClO}_4\text{)-Leu-}$$
$$\text{Arg(HClO}_4\text{)-Asp(OBu}^t\text{)-Ser(Bu}^t\text{)-Ala-Arg(HClO}_4\text{)-Leu-Gln-Arg}$$
$$\text{(HClO}_4\text{)-Leu-Leu-Gln-Gly-Leu-Val-NH}_2 \quad \text{HClO}_4 \qquad \text{(X)}$$

ist wie bei den bereits oben beschriebenen Benzyloxycarbonyl-Segmenten in Dimethylacetamid möglich. 1 g der Substanz IX lösen sich glatt in 10 ml Dimethylacetamid. Für 1 g des entsprechenden Tetrahydrobromids brauchte man zur Lösung etwa 140 ml einer Mischung aus Methanol/Dimethylacetamid wie 4:1 (Chem. Ber. 107, 1974, Seite 231) oder etwa 270 ml 80-prozentiger Essigsäure (Chem. Ber. 105, 1972, Seite 2512).

Die Umsetzung von X mit

Boc-His-Ser(Bu$^t$)-Asp(OBu$^t$)-Gly-Thr(Bu$^t$)-Phe-OH

zu Ib benötigt pro mmol etwa 40 ml einer Mischung aus Dimethylformamid und Dimethylacetamid (1:1). Mit dem entsprechenden Pentahydrobromid braucht man mehr als die doppelte Lösungsmittelmenge (Chem. Ber. 107, 1974, Seite 231) und unter Verwendung von DCC/N-Hydroxysucciniimid als Kondensationsreagenz wird etwa die fünffache Lösungsmittelmenge verbraucht (Chem. Ber. 105, 1972, Seite 2513).

Als Lösungsmittel für die katalytische Hydrierung der Benzyloxycarbonyl-Segmente, die unter Verwendung eines Autotitrators bei pH 4 - 6 durchgeführt wird, dient bevorzugt Dimethylacetamid, da Perchlorsäurelösungen in Dimethylacetamid stabiler sind als in Dimethylformamid. Der Formylrest wird durch Perchlorsäure unter Bildung von Dimethylamin leicht abgespalten. Auch Dimethylacetamid wird langsam von Perchlorsäure angegriffen. Verwendete Perchlorsäure/Dimethylacetamid-Lösungen sollten also nur frisch hergestellt werden. Doch kann man auch, wenn Wasser die Löslichkeit nicht zu stark herabsetzt, wäßrige Perchlorsäure (1-2 N) zur Titration der frei werdenden Aminogruppen heranziehen. Nach beendeter Hydrierung wird der Katalysator (Pd-Katalysator auf Kohle oder Bariumsulfat) abgesaugt und das Filtrat eingeengt. Der Rückstand kann dann mit geeigneten Lösungsmitteln, wie z.B. Essigester oder Diisopropylether verrieben werden, wobei sich meist amorphe Niederschläge bilden, die abgesaugt werden können. Auf der Stufe der Peptide VI und VIII empfiehlt sich eine Reinigung der Produkte. Das Dekapeptid-triperchlorat VI kann sehr gut durch Gegenstromverteilung zwischen n-Butanol und Wasser gereinigt werden, während das Tetradekapeptidtetraperchlorat VIII durch eine Gelfiltration an einem isopropylierten vernetzten Dextrangel in Wasser als Elutionsmittel gereinigt wird. Um Bakterienwuchs auf der Säule und im Eluat zu verhindern, wird Chloreton (1,1,1-Trichlor-2-methyl-2-propanol) bis zur Sättigung zugegeben. Das Chloreton kann nach der Reinigung durch Extraktion mit Essigester entfernt werden.

Die Aufarbeitung nach einer Segment-Kupplung mit DCC und HOObt ist einfach. Wenn möglich wird nach beendeter Reaktion der ausgefallene Dicyclohexylharnstoff abgesaugt und das Z-Peptid-perchlorat unter Zusatz der berechneten Menge Perchlorsäure mit Wasser ausgefällt. Fällt die Substanz schlecht filtrierbar aus, so verwandelt ein Zusatz von $NaClO_4$ die milchige Emulsion in eine gut filtrierbare Suspension.

Die Erfindung betrifft weiterhin ein Peptid der Formel I, in der R und $R^1$ die oben definierte Bedeutung haben.

Beispiel 1:

H-Arg($HClO_4$)-Leu-Leu-Gln-Gly-Leu-Val-$NH_2$ $HClO_4$ 22 g (18,2 mmol) Z-Arg($Z_2$)-Leu-Leu-Gln-Gly-Leu-Val-$NH_2$ werden in 220 ml Dimethylacetamid suspendiert. Nach Zugabe von Pd/Kohle-Katalysator wird am Autotitator unter Zugabe von 1N $HClO_4$ in Dimethylacetamid bei pH 4,5 katalytisch hydriert. (Die 1N $HClO_4$ in Dimethylacetamid sollte jeweils frisch hergestellt werden, da die Perchlorsäure Dimethylacetamid langsam zu Dimethylamin und Essigsäure hydrolysiert.)

Herstellung von ca. 1N $HClO_4$ in Dimethylacetamid: Zu 800 ml gut gekühltem Dimethylacetamid läßt man langsam unter Rühren 110 ml 60-proz. $HClO_4$ zutropfen (starke Wärmetönung) und füllt dann auf 1000 ml mit Dimethylacetamid auf.

Nach beendigter Hydrierung wird der Katalysator abgesaugt und das Filtrat auf etwa 100 ml eingeengt. Mit 600 ml Essigester wird das Peptid gefällt und abgesaugt. Anschließend wird das Peptid, das sehr hygroskopisch ist, noch einmal mit 600 ml Essigester verrührt, abgesaugt und im Hochvakuum über $P_2O_5$ getrocknet. Ausbeute 18,2 g (92%), Schmp. wird schaumig bei 112 - 124°, $[\alpha]_D^{20}$ = -30,7° (c = 1, 80-proz. Essigsäure)

$^1$H-NMR-Spektrum:     Bei $\delta$ = 2,7 - 2,9 sieht man die zwei Dimethylamid-Banden. Zwischen $\delta$ = 1,9 und 2,0 sind die Acetylgruppen des Dimethylacetamids und des Essigesters zu sehen.

Elementaranalyse:

| $C_{36}H_{68}N_{12}O_8 \cdot 2\ HClO_4 \cdot 0,8\ Dimethylacetamid \cdot 1\ H_2O$ (MG. 1085, 68) | | | | | |
|---|---|---|---|---|---|
| | C 43,37 | H 7,35 | Cl 6,53 | N 16,51 | $H_2O$ 1,66 |
| Ber. | | | | | |
| Gef. | 43,3 | 7,1 | 6,5 | 16,1 | 1,7 |

Beispiel 2:

Z-Arg($Z_2$)-Leu-Gln-Arg($HClO_4$)-Leu-Leu-Gln-Gly-Leu-Val-$NH_2$ Zu einer Lösung von 2,445 g (15 mmol) 3-Hydroxy-4-oxo-3,4-dihydro-1,2,3-benzotriazin (= HOObt) in 105 ml Dimethylacetamid gibt man bei 40°C unter Rühren 12,24 g (15 mmol) Z-Arg($Z_2$)-Leu-Gln-OH (feinzerrieben). Nachdem alles gelöst ist, läßt man auf Raumtemperatur kommen und gibt unter Rühren langsam 16,93 g (15 mmol) H-Arg-Leu-Leu-Gln-Gly-Leu-Val-$NH_2 \cdot 2\ HClO_4 \cdot 1\ H_2O \cdot 1,3$ DMA zu. Man rührt bei Raumtemperatur bis alles gelöst ist. Nun kühlt man auf 10°C ab (Lösung beginnt dick zu werden) und gibt nacheinander unter Rühren 1,95 ml (15 mmol) N-Ethylmorpholin und 3,9 g (18,9 mmol) Dicyclohexylcarbodiimid zu. Man läßt unter Rühren auf Raumtemperatur kommen. Normalerweise wird der Ansatz nach ca. 4 Stunden fest. In einzelnen Fällen bleibt der Ansatz bis zum nächsten Tag noch rührbar. Man läßt über Nacht bei Raumtemperatur stehen und verrührt den Ansatz mit einer Mischung aus 600 ml Eiswasser und 15 ml 2N wäßriger Perchlorsäure. Man rührt in der Kälte 15 Minuten und saugt ab. Man wäscht mit Wasser nach. Der Niederschlag wird darauf noch einmal mit 300 ml Wasser 1 - 2 Stunden bei Raumtemperatur verrührt. Der Niederschlag wird abgesaugt und mit Wasser gewaschen. Über $P_2O_5$ wird auf Gewichtskonstanz getrocknet.

Ausbeute: 28 g (92%), Schmp. 222°C (in anderen Ansätzen wurden auch Schmelzpunkte von 240°C oder 253 - 255°C gefunden).

$[\alpha]_D^{20}$ = -27,1° (c = 1, 80-proz. Essigsäure) (Drehwerte aus anderen Ansätzen: -27,8°, -28,9°, -27,4°).

Lt. Aufarbeitung enthält die Substanz 1 Moläquivalent Dicyclohexylharnstoff.

Lt. NMR enthält die Substanz 0,5 Moläquivalent Dimethylacetamid und laut Wasserbestimmung nach Fischer 3 Moläquivalent Wasser.

| Berechnetes Molekulargewicht: | |
|---|---|
| Peptid-perchlorat | 1697,40 |
| Dicyclohexylharnstoff | 224,34 |
| 3 Wasser | 54,048 |
| 0,5 Dimethylacetamid | 43,562 |
| Gesamtmolgewicht | 2019,35 |

Beispiel 3:

H-Arg(HClO$_4$)-Leu-Gln-Arg(HClO$_4$)-Leu-Leu-Gln-Gly-Leu-Val-NH$_2$ • HClO$_4$

27 g (13,37 mmol) Z-Arg(Z$_2$)-Leu-Gln-Arg-Leu-Leu-Gln-Gly-Leu-Val-NH$_2$ • HClO$_4$ • DC-Harnstoff • 3H$_2$O • 0,5 Dimethylacetamid werden in 300 ml Dimethylacetamid suspendiert. Nach Zugabe von Pd/Kohle-Katalysator wird am Autotitrator unter Zugabe von frisch hergestellter 1N HClO$_4$ in Dimethylacetamid bei pH 4,5 katalytisch hydriert. Nach beendigter Hydrierung wird der Katalysator abgesaugt und das Filtrat vollständig im Hochvakuum eingeengt. Der Rest wird in 150 ml mit n-Butanol gesättigter wäßriger 0,02 N HCl gelöst. Von unlöslichen Bestandteilen (hauptsächlich DC-Harnstoff) wird filtriert und anschließend in 7 Stufen zwischen 0,02 N HCl und n-Butanol gegenstromverteilt. Die n-Butanolphase soll mit 0,02 N HCl und die 0,02 N HCl soll mit n-Butanol gesättigt sein. Die einzelnen Stufen werden im DC überprüft (DC: n-Butanol/Pyridin/Wasser/Eisessig wie 60/20/24/6). Die guten Fraktionen (Wasser 5 - 7 und n-Butanol 4 - 7) werden vereinigt und im Hochvakuum eingeengt. Der Rückstand wird mit Diisopropylether verrieben und abgesaugt. Die Substanz ist zunächst sehr hygroskopisch. Nach dem Trocknen über P$_2$O$_5$ im Vakuum verliert sich diese Eigenschaft. Ausbeute 15,15 g (Hauptfraktion). Die Nebenfraktionen (Wasser 3 - 4 und n-Butanol 2 - 3), die neben Verunreinigungen noch ansehnliche Mengen des gewünschten Peptids enthalten, werden getrennt eingeengt und nochmals wie oben in 100-ml-Phasenmengen gegenstromverteilt. Die brauchbaren Fraktionen (Wasser 3 - 7 und n-Butanol 7) werden eingeengt, mit Diisopropylether verrieben, abgesaugt und über P$_2$O$_5$ im Hochvakuum getrocknet. Um das störende Wasser zu entfernen, empfiehlt sich vor dem Verreiben mit Diisopropylether ein Nachdestillieren mit Toluol. Ausbeute 5,45 g (2. Fraktion). Gesamtausbeute 20,6 g (72%).

$[\alpha]_D^{20}$ = -27,6° (c = l, 80-proz. Essigsäure).

Die Substanz enthält laut NMR 6 Moläquivalente Dimethylacetamid, 0,75 Moläquivalente Diisopropylether und lt. Wasserbestimmung nach Fischer 1,5 Moläquivalente H$_2$O.

| Berechnetes Molgewicht: | |
|---|---|
| Dekapeptid • 3 HClO$_4$ | 1495,9 |
| 6 Dimethylacetamid | 522,7 |
| 0,75 Diisopropylether | 76,6 |
| 1,5 Wasser | 27,6 |
| Gesamtmolgewicht: | 2122,8 |

Beispiel 4:

Z-Arg(Z$_2$)-Asp(OBu$^t$)-Ser(Bu$^t$)-Ala-Arg(HClO$_4$)-Leu-Gln-Arg(HClO$_4$)-Leu-Leu-Gln-Gly-Leu-Val-NH$_2$

8,73 g (9 mmol) Z-Arg(Z$_2$)-Asp(OBu$^t$)-Ser(Bu$^t$)-Ala-OH • 0,5 H$_2$O und 1,47 g (9 mmol) 3-Hydroxy-4-oxo-3,4-dihydro-1,2,3-benzotriazin ( = HOObt) werden in 45 ml Dimethylacetamid gelöst. Unter Rühren gibt man 19,1 g (9 mmol) H-Arg-Leu-Gln-Arg-Leu-Leu-Gln-Gly-Leu-Val-NH$_2$·3HClO$_4$ (enthält lt. NMR 0,75 Moläquivalent Diisopropylether und 6 Moläquivalent Dimethylacetamid, sowie 1,5 Moläquivalent H$_2$O) zu und erwärmt auf 40°C, wobei alles in Lösung geht. Anschließend kühlt man auf 0°C ab und gibt 2,34 ml (18 mmol) N-Ethylmorpholin zu. Die Reaktionslösung sollte hierbei eine gelbe Farbe annehmen (HOObt wirkt als Indikator!). Tritt der Farbumschlag nicht ein, enthält das Dekapeptid von der Gegenstromverteilung her noch zu viel Salzsäure und man gibt vorsichtig weiteres N-Ethylmorpholin bis zum Farbumschlag ins Gelbe zu. (Überschüssiges N-Ethylmorpholin wegen Racemisierungsgefahr vermeiden !). Anschließend gibt man 2,34 g (11,26 mmol) DCC zu, rührt eine Stunde bei 0°C und drei Stunden bei Raumtemperatur. Man läßt über Nacht bei Raumtemperatur stehen. DC-Kontrolle auf vollständige Umsetzung in Eisessig/n-Butanol/Wasser wie 1:3:1.

Der Ansatz erstarrt zu einer gallertigen Masse, die mit 1000 ml Wasser unter Zusatz von 4,5 ml 2N wäßriger Perchlorsäure verrieben wird. Der Niederschlag wird abgesaugt, gut mit Wasser gewaschen und über P$_2$O$_5$ im Vakuum getrocknet.

Ausbeute 22,9 g (99%). Die Substanz enthält 1 Moläquivalent DC-Harnstoff. Schmp. 194° - 200°C

$[\alpha]_D^{20}$ = -22,6° (c = l, in 80-proz. Essigsäure)

Beispiel 5:

H-Arg(HClO$_4$)-Asp(OBu$^t$)-Ser(Bu$^t$)-Ala-Arg(HClO$_4$)-Leu-Gln-Arg(HClO$_4$)-Leu-Leu-Gln-Gly-Leu-Val-NH$_2$·HClO$_4$

37 g (14,4 mmol) Z-Arg(Z$_2$)-Arg(OBu$^t$)-Ser(Bu$^t$)-Ala-Arg-Leu-Gln-Arg-Leu-Leu-Gln-Gly-Leu-Val-NH$_2$·2 HClO$_4$ (+DC-Harnstoff) werden in 370 ml Dimethylacetamid gelöst. Die Lösung bleibt trüb. Ohne zu filtrieren versetzt man mit Pd-Katalysator und hydriert unter Zugabe von frisch bereiteter 1N HClO$_4$ in Dimethylacetamid oder Wasser am Autotitrator bei pH 4,5. Nach Beendigung der Hydrierung wird der Katalysator abgesaugt und das Filtrat eingeengt. Der Rückstand wird in 60 ml Wasser gelöst und von Unlöslichem (in der Hauptsache DC-Harnstoff) filtriert. Das Filtrat wird an Sephadex LH 20 chromatographiert. Als Eluens dient Wasser, das mit Chloreton gesättigt ist (Chloreton dient zur Desinfektion und kann später gut entfernt werden!)

Säulenabmessungen ca. 4 m lang und 8 cm Durchmesser. Wichtig ist die Abtrennung der ninhydrinpositiven Verunreinigungen (als Hauptverunreinigung konnte H-Arg-Asp (OBu$^t$)-Ser(Bu$^t$)-Ala-OH festgestellt werden). DC-Kontrolle des Eluats in n-Butanol/Pyridin/Wasser/Eisessig wie 60:20:24:6. Das peptidhaltige Eluat wird zur Entfernung des Chloretons dreimal mit Essigester extrahiert und nach Abdestillation des in Wasser gelöstem Essigester, gefriergetrocknet. Ausbeute 17,73 g (55%).

$[\alpha]_D^{20}$ = -26,8° (c = l, in 80-proz. Essigsäure).

Die Substanz ist lt. Analyse ein Hexahydrat.

Elementaranalyse:

| C$_{77}$H$_{146}$Cl$_4$N$_{26}$O$_{35}$·6 H$_2$O (2246,2) | | | | |
|---|---|---|---|---|
| Ber. | C 41,17 | H 7,09 | Cl 6,31 | N 16,21 | H$_2$O 4,82 |
| Gef. | 41,3 | 7,1 | 6,4 | 16,0 | 5,1 |

Beispiel 6:

Z-Thr(Bu$^t$)-Ser(Bu$^t$)-Glu(OBu$^t$)-Leu-Ser(Bu$^t$)-Arg(HClO$_4$)-Leu-Arg(HClO$_4$)-Asp(OBu$^t$)-Ser(Bu$^t$)-Ala-Arg(HClO$_4$)-Leu-Gln-Arg(HClO$_4$)-Leu-Leu-Gln-Gly-Leu-Val-NH$_2$

a) 1,2 g (1 mmol) Z-Thr(Bu$^t$)-Ser(Bu$^t$)-Glu(OBu$^t$)-Leu-Ser (Bu$^t$)-Arg-Leu-OH•2 H$_2$O und 163 mg (1 mmol) 3-Hydroxy-4-oxo-3,4-dihydro-1,2,3-benzotriazin (=HOObt) werden in einer Mischung aus 10 ml Dimethylacetamid und 10 ml Dimethylformamid suspendiert. Dazu gibt man bei Raumtemperatur 2,24 g (1 mmol) H-Arg-Asp(OBu$^t$)-Ser(Bu$^t$)-Ala-Arg-Leu-Gln-Arg-Leu-Leu-Gln-Gly-Leu-Val-NH$_2$•4 HClO$_4$•6 H$_2$O. Dabei tritt Lösung ein. Nun gibt man 0,26 ml (2 mmol) N-Ethylmorpholin und eine Lösung von 440 mg (2,13 mmol) Dicyclohexylcarbodiimid (=DCC) in 5 ml Dimethylacetamid bei Raumtemperatur zu. Man läßt etwa 4 Stunden bei Raumtemperatur rühren und über Nacht bei Raumtemperatur stehen. Wenn möglich (falls die Lösung nicht gallertig geworden ist) saugt man anderntags vom DC-Harnstoff ab und verrührt das Filtrat mit 200 ml Wasser, dem vorher 1 ml 2N HClO$_4$ (2 mmol) zugesetzt wird. Das Peptid fällt emulsionsartig aus. Durch Zusatz von 4 ml einer 50-proz. wäßrigen NaClO$_4$-Lösung flockt das Peptid gut aus. Es wird abgesaugt und mit wenig Wasser nachgewaschen.

Ausbeute nach Trocknen über P$_2$O$_5$: 3,17 g (96,5%)

$[\alpha]_D^{20}$ = -17,8° (c=l, in 80-proz. Essigsäure)

b) Zu einer Suspension von 600 mg (0,5 mmol) Z-Thr(Bu$^t$)-Ser(Bu$^t$)-Glu(OBu$^t$)-Leu-Ser(Bu$^t$)-Arg-Leu-OH•2 H$_2$O in 0,5 ml Dimethylacetamid gibt man 90 mg (0,5 mmol) Pyridiniumperchlorat und 81,5 mg (0,4 mmol) HOObt. Nachdem alles gelöst ist, gibt man 412 mg (2 mmol) DCC zu, läßt zwei Stunden bei Raumtemperatur rühren. Inzwischen werden 1,07 g (0,5 mmol) H-Arg-Asp(OBu$^t$)-Ser(Bu$^t$)-Ala-Arg-Leu-Gln-Arg-Leu-Leu-Gln-Gly-Leu-Val-NH$_2$•4 HClO$_4$• 6 H$_2$O in 5 ml Dimethylacetamid gelöst. In diese Lösung saugt man über ein kleines Filter obigen Ansatz, wäscht mit wenig Dimethylacetamid nach, gibt noch 0,13 ml (1 mmol) N-Ethylmorpholin zu und läßt 2 Stunden bei Raumtemperatur rühren. Danach wird durch Zugabe von 100 ml Wasser das Peptid ausgefällt. Das Peptid wird abzentrifugiert und getrocknet,

Ausbeute: 1,21 g.

Beispiel 7:

$$\text{H-Thr(Bu}^t\text{)-Ser(Bu}^t\text{)-Glu(OBu}^t\text{)-Leu-Ser(Bu}^t\text{)-Arg(HClO}_4\text{)-Leu-}$$
$$\text{Arg(HClO}_4\text{)-Asp(OBu}^t\text{)-Ser(Bu}^t\text{)-Ala-Arg(HClO}_4\text{)-Leu-Gln-Arg}$$
$$\text{(HClO}_4\text{)-Leu-Leu-Gln-Gly-Leu-Val-NH}_2\text{•HClO}_4$$

3,3 g (ca. 1 mmol) geschütztes Z-Sekretin-(7-27)-heneikosapeptidtetraperchlorat (Formel siehe oben!) werden in 33 ml Dimethylacetamid gelöst, mit Pd/Kohle-Katalysator versetzt und unter Zugabe von frisch bereiteter 1N HClO$_4$ in Dimethylacetamid oder Wasser am Autotitrator bei pH 4,5 katalytisch hydriert. Nach beendeter Reaktion wird der Katalysator abgesaugt und das Filtrat im Hochvakuum eingeengt. Der Rückstand wird mit Essigester verrieben und abgesaugt.

Ausbeute 3,17 g (97%)

$[\alpha]_D^{20}$ = -22,0° (c=l, in 80-proz. Essigsäure)

Beispiel 8:

$$\text{Boc-His-Ser(Bu}^t\text{)-Asp(OBu}^t\text{)-Gly-Thr(Bu}^t\text{)-Phe-Thr(Bu}^t\text{)-}$$
$$\text{Ser(Bu}^t\text{)-Glu(OBu}^t\text{)-Leu-Ser(Bu}^t\text{)-Leu-Ser(Bu}^t\text{)-Arg(HClO}_4\text{)-}$$
$$\text{Leu-Arg(HClO}_4\text{)-Asp(OBu}^t\text{)-Ser(Bu}^t\text{)-Ala-Arg(HClO}_4\text{)-Leu-Gln-}$$
$$\text{Arg(HClO}_4\text{)-Leu-Leu-Gln-Gly-Leu-Val-NH}_2$$

Zu einer Lösung von 9,51 g Boc-His-Ser(Bu$^t$)-Asp(OBu$^t$)-Gly-Thr(Bu$^t$)-Phe-OH und 1,36 g HOObt in 166 ml Dimethylacetamid und 166 ml Dimethylformamid gibt man unter Rühren 27,2 g (8,3 mmol)

$$H-Thr(Bu^t)-Ser(Bu^t)-Glu(OBu^t)-Leu-Ser(Bu^t)-Arg(HClO_4)-Leu-$$
$$Arg(HClO_4)-Asp(OBu^t)-Ser(Bu^t)-Ala-Arg(HClO_4)-Leu-Gln-Arg$$
$$(HClO_4)-Leu-Leu-Gln-Gly-Leu-Val-NH_2 \cdot HClO_4.$$

Nachdem alles gelöst ist, gibt man bei 0°C 2,2 ml N-Ethylmorpholin und 3,7 g Dicyclohexylcarbodiimid zu, läßt eine Stunde bei 0° und über Nacht bei Raumtemperatur rühren. Anderntags wird die Lösung in eine Mischung aus 1,3 Liter Wasser, 8,3 ml 2N $HClO_4$ und 42,5 ml einer 50-proz. $NaClO_4$-Lösung eingesaugt. Der Niederschlag wird abgesaugt, mit Wasser gewaschen und im Vakuum über $P_2O_5$ getrocknet.

Ausbeute 32,9 g.

Laut Aminosäureanalyse ist der Gehalt an Peptid etwa 80% (= 83% Ausbeute).

$[\alpha]_D^{20}$ = -11,8° (c = l, in 80-proz. Essigsäure).

Beispiel 9:

a) Fmoc-Leu-OObt

Zu einer Lösung von 3,53 g Fmoc-Leu-OH und 1,63 g HOObt in 40 ml Methylenchlorid gibt man bei 0°C 2,06 g DCC. Man rührt 1 Stunde bei 0°C und läßt über Nacht bei Raumtemperatur stehen. Der Niederschlag wird abgesaugt und das Filtrat eingeengt. Der Rückstand wird zwei mal mit Petrolether verrieben, abgesaugt und im Vakuum getrocknet. Ausbeute 5,2 g, amorph.

b) Fmoc-Leu-Arg-OH

Zu einer Suspension von 1,74 g L-Arginin, 1,63 g HOObt und 1,8 g Pyridiniumperchlorat in 20 ml Dimethylacedamid gibt man 5 g Fmoc-Leu-OObt und rührt bei Raumtemperatur. Nach 1 Stunde ist alles gelöst. Man läßt über Nacht bei Raumtemperatur stehen und engt anderntags ein. Der Rückstand wird zwischen 180 ml Pentanol und 160 ml Wasser (+ 20 ml gesättigter wäßriger $NaHCO_3$-Lösung) verteilt. Dieser Verteilung schließt man eine 5-stufige Gegenstromverteilung zwischen je 180 ml Pentanol und 180 ml Wasser an. Die Fraktionen mit sauberem Fmoc-Leu-Arg-OH werden eingeengt und der Rückstand mit Ether verrieben.

Ausbeute: 4,1 g, Schmp. 145 - 155°C unter Zersetzung,

$[\alpha]_D^{20}$ = -18,5° (c = l, in Methanol).

Beispiel 10:

H-Asn-Ser-Phe-Arg-Tyr-OH

Die Synthese wurde mit einem "Applied Biosystems 430 A peptide synthesizer" durchgeführt.

Der Aufbau des Peptids erfolgte an einem p-Benzyloxybenzylalkohol-Harz ("Wang-Harz": S. Wang, J. Am. Chem. Soc. 95, 1328 (1973)), das nach bekannter Methode (E. Atherton et al., J. Chem. Soc. Chem. Comm. 1981, 336) mit Fmoc-Tyr (Bu$^t$)-OH verestert wurde (Substitutionsgrad: 0,4 mequiv./g). Es wurde 1 g Harz für die Synthese eingesetzt. In die vom Gerätehersteller gelieferten Cartridges wurden je 1 mmol Fmoc-Asn-OH, Fmoc-Ser(Bu$^t$)-OH, Fmoc-Phe-OH, Fmoc-Arg-OH zusammen mit 1,5 - 2,5 Äquivalenten HOBt eingewogen. Im Falle des Argininderivats wurde 1 Äquivalent Pyridiniumperchlorat zugefügt. Die Aktivierung der Aminosäurederivate als HOBt-Ester erfolgte in den Cartridges durch Lösen in 4 ml Dimethylformamid und anschließender Zugabe von 2 ml einer 0,55 M Lösung von Diisopropylcarbodiimid in Dimethylformamid.

Ein typischer Synthesezyklus ist nachfolgend aufgelistet:

1. Abspaltung der Schutzgruppe mit 2 x 8 ml einer 20-proz. Lösung von Piperidin in Dimethylformamid für jeweils 10 Minuten.

2. Waschen mit Dimethylformamid (6 x 8 ml).

3. Vorbeladen des Harzes mit HOBt durch 10-min. Behandeln mit einer 0,5 M Lösung von HOBt In Dimethylformamid und anschließendes Abpumpen der Lösung.

4. Kupplung des in der Cartridge 30 - 45 min voraktivierten Fmoc-Aminosäure-OBt-esters für 25 - 45 min.

5. Waschen mit Dimethylformamid (6 x 8 ml).

Nach beendeter Synthese wird vom Peptid-Harz zunächst mit Hilfe von 20-proz. Piperidin in Dimethylformamid die Fmoc-Gruppe abgespalten und dann von einer Probe (100 mg) durch zweistündiges Behandeln mit Trifluoressigsäure/Methylenchlorid/Phenol wie 70:30:5 das Peptid abgespalten. Das Harz wird abfiltriert, mit Abspaltlösung nachgewaschen und das Filtrat im Vakuum eingeengt. Durch mehrfaches Verrühren mit Essigester wird das Phenol entfernt. Das verbleibende Rohpeptid wird in 10-proz. Essigsäure gelöst, filtriert und die Lösung gefriergetrocknet.

Ausbeute: 23,8 mg Rohpeptid;

Aminosäureanalyse nach Hydrolyse in 6N HCl bei 120°C für 24 Stunden: Asp (1,03), Ser (0,81), Tyr (0,89), Phe (1,00), Arg (0,88), kein Ornithin (Ornithin entsteht aus an der Guandinogruppe acyliertem Arginin bei saurer Hydrolyse.). FAB-Massenspektrum: 686 (= Molmasse + $H^+$).

**Patentansprüche**

**Patentansprüche für folgende Vertragsstaaten : BE, CH, DE, FR, GB, GR, IT, LI, LU, NL, SE**

1. Verfahren zur Herstellung von geschützten argininhaltigen Peptiden durch Fragmentkupplung, dadurch gekennzeichnet, daß man bei der jeweiligen Fragmentkupplung mindestens ein argininhaltiges Fragment als Perchlorat umsetzt.

2. Verfahren gemäß Anspruch 1, dadurch gekennzeichnet, daß man ein gegebenenfalls geschütztes Arginin als Perchlorat umsetzt.

3. Verfahren gemäß Anspruch 1 oder 2, dadurch gekennzeichnet, daß man ein argininhaltiges Segment als Perchlorat umsetzt.

4. Verfahren gemäß einem der Ansprüche 1 - 3, dadurch gekennzeichnet, daß ein Fragment während des Syntheseablaufs an einem Festkörper kovalent gebunden ist.

5. Verfahren gemäß einem der Ansprüche 1 - 4, dadurch gekennzeichnet, daß man die Kupplung in Gegenwart eines Carbodiimids durchführt.

6. Verfahren gemäß einem der Ansprüche 1 - 5, dadurch gekennzeichnet, daß man die Kupplung in Gegenwart von N, N'-Diisopropylcarbodiimid oder N, N'-Dicyclohexylcarbodiimid gegebenenfalls unter Zusatz von N-Hydroxysuccinimid, 1-Hydroxybenzotriazol, 3-Hydroxy-4-oxo-3,4-dihydro-1,2,3-benzotriazinoder anderer racemisierungssenkender Verbindungen durchführt.

7. Verfahren gemäß einem der Ansprüche 1 - 3 zur Herstellung eines geschütztes Sekretinderivats der Formel I,

$$R-His(R^1)-Ser(Bu^t)-Asp(OBu^t)-Gly-Thr(Bu)^t-Phe-Thr(Bu^t)-$$
$$Ser-Bu^t)-Glu(OBu^t)-Leu-Ser(Bu^t)-Arg(HClO_4)-Leu-Arg(HClO_4)-$$

$$Asp(OBu^t)-Ser(Bu^t)-Ala-Arg(HClO_4)-Leu-Gln-Arg(HClO_4)-Leu-$$
$$Leu-Gln-Gly-Leu-Val-NH_2 \qquad (I)$$

in der

a) R und $R^1$ den Boc-Rest oder

b) R den Boc-Rest und $R^1$ Wasserstoff oder

c) R und $R^1$ den Adamantyloxycarbonyl-Rest bedeuten,

dadurch gekennzeichnet, daß man ein Peptid der allgemeinen Formel IIa,

H-X-Leu-Leu-Gln-Gly-Leu-Val-NH$_2$ (IIa)

in der X

$$-Arg(HClO_4)-,$$
$$-Arg(HClO_4)-Leu-Gln-Arg(HClO_4)-,$$
$$-Arg(HClO_4)-Asp(OBu^t)-Ser(Bu^t)-Ala-Arg(HClO_4)-Leu-Gln-$$
$$Arg(HClO_4)-$$

oder

$$-Thr(Bu^t)-Ser(Bu^t)-Glu(OBu^t)-Leu-Ser(Bu^t)-Arg(HClO_4)-Leu-$$
$$Arg(HClO_4)-Asp(OBu^t)-Ser(Bu^t)-Ala-Arg(HClO_4)-Leu-Gln-Arg$$
$$(HClO_4)-$$

bedeutet, unter Zusatz von 3-Hydroxy-4-oxo-3,4-dihydro-1,2,3-benzotriazin mit den entsprechenden Peptiden mit freier Carboxylgruppe in polaren Lösungsmitteln mit Dicyclohexylcarboiimid und notwendigen Mengen einer tertiären Base umsetzt und die so synthetisierten Peptide aus Wasser unter Zusatz entsprechender Mengen Perchlorsäure und gegebenenfalls eines Perchlorats als Perchlorate ausfällt.

8. Verfahren gemäß Anspruch 7, dadurch gekennzeichnet, daß das Peptid der allgemeinen Formel IIa durch Hydrierung benzyloxycarbonylhaltiger Fragmente der allgemeinen Formel II,

Z-X-Leu-Leu-Gln-Gly-Leu-Val-NH$_2$ (II)

in der X

$$-Arg(Z_2)-$$
$$-Arg(Z_2)-Leu-Gln-Arg(HClO_4)-$$
$$-Arg(Z_2)-Asp(OBu^t)-Ser(Bu^t)-Ala-Arg(HClO_4)-Leu-Gln-$$
$$Arg(HClO_4)-$$

oder

$$-Thr(Bu^t)-Ser(Bu^t)-Glu(OBu^t)-Leu-Ser(Bu^t)-Leu-Ser(Bu^t)-$$
$$Arg(HClO_4)-Leu-Arg(HClO_4)-Asp(OBu^t)-Ser(Bu^t)-Ala-Arg$$
$$(HClO_4)-Leu-Gln-Arg(HClO_4)-$$

bedeutet, in Dimethylacetamid unter Zusatz eines Palladiumkatalysators, wobei durch Zugabe einer perchlorsäurehaltigen Lösung der pH-Wert zwischen 4 und 6 gehalten wird, erhalten und weiter gemäß Anspruch 7 umgesetzt wird.

9. Verfahren gemäß einem der Ansprüche 1 - 8, dadurch gekennzeichnet, daß man Pyridiniumperchlorat zur Herstellung des Perchlorats des argininhaltigen Fragments verwendet.

**10.** Peptid der Formel I, in der R und R[1] die im Anspruch 7 definierten Bedeutungen haben.

**Patentansprüche für folgende Vertragsstaaten : AT, ES**

**1.** Verfahren zur Herstellung von geschützten argininhaltigen Peptiden durch Fragmentkupplung, dadurch gekennzeichnet, daß man bei der jeweiligen Fragmentkupplung mindestens ein argininhaltiges Fragment als Perchlorat umsetzt.

**2.** Verfahren gemäß Anspruch 1, dadurch gekennzeichnet, daß man ein gegebenenfalls geschütztes Arginin als Perchlorat umsetzt.

**3.** Verfahren gemäß Anspruch 1 oder 2, dadurch gekennzeichnet, daß man ein argininhaltiges Segment als Perchlorat umsetzt.

**4.** Verfahren gemäß einem der Ansprüche 1 - 3, dadurch gekennzeichnet, daß ein Fragment während des Syntheseablaufs an einem Festkörper kovalent gebunden ist.

**5.** Verfahren gemäß einem der Ansprüche 1 - 4, dadurch gekennzeichnet, daß man die Kupplung in Gegenwart eines Carbodiimids durchführt.

**6.** Verfahren gemäß einem der Ansprüche 1 - 5, dadurch gekennzeichnet, daß man die Kupplung in Gegenwart von N, N'-Diisopropylcarbodiimid oder N, N'-Dicyclohexylcarbodiimid gegebenenfalls unter Zusatz von N-Hydroxysuccinimid, 1-Hydroxybenzotriazol, 3-Hydroxy-4-oxo-3,4-dihydro-1,2,3-benzotriazinoder anderer racemisierungssenkender Verbindungen durchführt.

**7.** Verfahren gemäß einem der Ansprüche 1 - 3 zur Herstellung eines geschütztes Sekretinderivats der Formel I,

$$R-His(R^1)-Ser(Bu^t)-Asp(OBu^t)-Gly-Thr(Bu)^t-Phe-Thr(Bu^t)-$$
$$Ser-Bu^t)-Glu(OBu^t)-Leu-Ser(Bu^t)-Arg(HClO_4)-Leu-Arg(HClO_4)-$$

$$Asp(OBu^t)-Ser(Bu^t)-Ala-Arg(HClO_4)-Leu-Gln-Arg(HClO_4)-Leu-$$
$$Leu-Gln-Gly-Leu-Val-NH_2 \qquad\qquad (I)$$

in der
a) R und R[1] den Boc-Rest oder
b) R den Boc-Rest und R[1] Wasserstoff oder
c) R und R[1] den Adamantyloxycarbonyl-Rest bedeuten,
dadurch gekennzeichnet, daß man ein Peptid der allgemeinen Formel IIa,

H-X-Leu-Leu-Gln-Gly-Leu-Val-NH$_2$ (IIa)

in der X

$$-Arg(HClO_4)-,$$
$$-Arg(HClO_4)-Leu-Gln-Arg(HClO_4)-,$$
$$-Arg(HClO_4)-Asp(OBu^t)-Ser(Bu^t)-Ala-Arg(HClO_4)-Leu-Gln-$$
$$Arg(HClO_4)-$$

oder

$$-\text{Thr}(\text{Bu}^t)-\text{Ser}(\text{Bu}^t)-\text{Glu}(\text{OBu}^t)-\text{Leu}-\text{Ser}(\text{Bu}^t)-\text{Arg}(\text{HClO}_4)-\text{Leu}-$$
$$\text{Arg}(\text{HClO}_4)-\text{Asp}(\text{OBu}^t)-\text{Ser}(\text{Bu}^t)-\text{Ala}-\text{Arg}(\text{HClO}_4)-\text{Leu}-\text{Gln}-\text{Arg}$$
$$(\text{HClO}_4)-$$

bedeutet, unter Zusatz von 3-Hydroxy-4-oxo-3.4-dihydro-1.2.3-benzotriazin mit den entsprechenden Peptiden mit freier Carboxylgruppe in polaren Lösungsmitteln mit Dicyclohexylcarboiimid und notwendigen Mengen einer tertiären Base umsetzt und die so synthetisierten Peptide aus Wasser unter Zusatz entsprechender Mengen Perchlorsäure und gegebenenfalls eines Perchlorats als Perchlorate ausfällt.

**8.** Verfahren gemäß Anspruch 7, dadurch gekennzeichnet, daß das Peptid der allgemeinen Formel IIa durch Hydrierung benzyloxycarbonylhaltiger Fragmente der allgemeinen Formel II,

Z-X-Leu-Leu-Gln-Gly-Leu-Val-NH$_2$ (II)

in der X

$$-\text{Arg}(\text{Z}_2)-$$
$$-\text{Arg}(\text{Z}_2)-\text{Leu}-\text{Gln}-\text{Arg}(\text{HClO}_4)-$$
$$-\text{Arg}(\text{Z}_2)-\text{Asp}(\text{OBu}^t)-\text{Ser}(\text{Bu}^t)-\text{Ala}-\text{Arg}(\text{HClO}_4)-\text{Leu}-\text{Gln}-$$
$$\text{Arg}(\text{HClO}_4)-$$

oder

$$-\text{Thr}(\text{Bu}^t)-\text{Ser}(\text{Bu}^t)-\text{Glu}(\text{OBu}^t)-\text{Leu}-\text{Ser}(\text{Bu}^t)-\text{Leu}-\text{Ser}(\text{Bu}^t)-$$
$$\text{Arg}(\text{HClO}_4)-\text{Leu}-\text{Arg}(\text{HClO}_4)-\text{Asp}(\text{OBu}^t)-\text{Ser}(\text{Bu}^t)-\text{Ala}-\text{Arg}$$
$$(\text{HClO}_4)-\text{Leu}-\text{Gln}-\text{Arg}(\text{HClO}_4)-$$

bedeutet, in Dimethylacetamid unter Zusatz eines Palladiumkatalysators, wobei durch Zugabe einer perchlorsäurehaltigen Lösung der pH-Wert zwischen 4 und 6 gehalten wird, erhalten und weiter gemäß Anspruch 7 umgesetzt wird.

**9.** Verfahren gemäß einem der Ansprüche 1 - 8, dadurch gekennzeichnet, daß man Pyridiniumperchlorat zur Herstellung des Perchlorats des argininhaltigen Fragments verwendet.

**Claims**
**Claims for the following Contracting States : BE, CH, DE, FR, GB, GR, IT, LI, LU, NL, SE**

**1.** A process for the preparation of protected arginine-containing peptides by fragment coupling, which comprises in each fragment coupling at least one arginine-containing fragment being reacted as perchlorate.

**2.** The process as claimed in claim 1, wherein an optionally protected arginine is reacted as perchlorate.

**3.** The process as claimed in claim 1 or 2, wherein an arginine-containing segment is reacted as perchlorate.

**4.** The process as claimed in one of claims 1 - 3, wherein one fragment is covalently bonded to a solid phase during the course of the synthesis.

5. The process as claimed in one of claims 1 - 4, wherein the coupling is carried out in the presence of a carbodiimide.

6. The process as claimed in one of claims 1 - 5, wherein the coupling is carried out in the presence of N,N'-diisopropylcarbodiimide or N,N'-dicyclohexylcarbodiimide, where appropriate with the addition of N-hydroxysuccinimide, 1-hydroxybenzotriazole, 3-hydroxy-4-oxo-3,4-dihydro-1,2,3-benzotriazine or other racemization-reducing compounds.

7. The process as claimed in one of claims 1 - 3 for the preparation of a protected secretin derivative of the formula I

$$R-His(R^1)-Ser(Bu^t)-Asp(OBu^t)-Gly-Thr(Bu^t)-Phe-$$
$$Thr(Bu^t)-Ser(Bu^t)-Glu(OBu^t)-Leu-Ser(Bu^t)-Arg(HClO_4)-$$
$$Leu-Arg(HClO_4)-Asp(OBu^t)-Ser(Bu^t)-Ala-Arg(HClO_4)-Leu-$$
$$Gln-Arg(HClO_4)-Leu-Leu-Gln-Gly-Leu-Val-NH_2$$

$$(I)$$

in which
  a) R and $R^1$ denote the Boc radical or
  b) R denotes the Boc radical, and $R^1$ denotes hydrogen or
  c) R and $R^1$ denote the adamantyloxycarbonyl radical,
which comprises reaction of a peptide of the general formula IIa

H-X-Leu-Leu-Gln-Gly-Leu-Val-NH$_2$ (IIa)

in which X denotes

$$-Arg(HClO_4)-,$$
$$-Arg(HClO_4)-Leu-Gln-Arg(HClO_4)-,$$
$$-Arg(HClO_4)-Asp(OBu^t)-Ser(Bu^t)-Ala-Arg(HClO_4)-$$
$$\ \ Leu-Gln-Arg(HClO_4)-$$

or

$$-Thr(Bu^t)-Ser(Bu^t)-Glu(OBu^t)-Leu-Ser(Bu^t)-Arg(HClO_4)-$$
$$Leu-Arg(HClO_4)-Asp(OBu^t)-Ser(Bu^t)-Ala-Arg(HClO_4)-Leu-$$
$$Gln-Arg(HClO_4)-$$

with the addition of 3-hydroxy-4-oxo-3,4-dihydro-1,2,3-benzotriazine with the appropriate peptides with free carboxyl group in polar solvents with dicyclohexylcarbodiimide and the necessary amounts of a tertiary base, and precipitation of the peptides, which have thus been synthesized, as perchlorates from water with the addition of appropriate amounts of perchloric acid and, where appropriate, of a perchlorate.

8. The process as claimed in claim 7, wherein the peptide of the general formula IIa is obtained by hydrogenation of benzyloxycarbonyl-containing fragments of the general formula II

Z-X-Leu-Leu-Gln-Gly-Leu-Val-NH$_2$ (II)

in which X denotes

$$-Arg(Z_2)-$$
$$-Arg(Z_2)-Leu-Gln-Arg(HClO_4)-$$
$$-Arg(Z_2)-Asp(OBu^t)-Ser(Bu^t)-Ala-Arg(HClO_4)-Leu-Gln-$$
$$Arg(HClO_4)-$$

or

$$-Thr(Bu^t)-Ser(Bu^t)-Glu(OBu^t)-Leu-Ser(Bu^t)-Leu-$$
$$Ser(Bu^t)-Arg(HClO_4)-Leu-Arg(HClO_4)-Asp(OBu^t)-$$
$$Ser(Bu^t)-Ala-Arg(HClO_4)-Leu-Gln-Arg(HClO_4)-$$

in dimethylacetamide with the addition of a palladium catalyst, the pH being maintained between 4 and 6 by addition of a solution containing perchloric acid, and is reacted further as claimed in claim 7.

9. The process as claimed in one of claims 1 - 8, wherein pyridinium perchlorate is used for the preparation of the perchlorate of the arginine-containing fragment.

10. A peptide of the formula I in which R and R$^1$ have the meanings defined in claim 7.

**Claims for the following Contracting States : AT, ES**

1. A process for the preparation of protected arginine-containing peptides by fragment coupling, which comprises in each fragment coupling at least one arginine-containing fragment being reacted as perchlorate.

2. The process as claimed in claim 1, wherein an optionally protected arginine is reacted as perchlorate.

3. The process as claimed in claim 1, wherein an arginine-containing segment is reacted as perchlorate.

4. The process as claimed in claim 1, wherein one fragment is covalently bonded to a solid phase during the course of the synthesis.

5. The process as claimed in one of claims 1 - 4, wherein the coupling is carried out in the presence of a carbodiimide.

6. The process as claimed in one of claims 1 - 5, wherein the coupling is carried out in the presence of N,N'-diisopropylcarbodiimide or N,N'-dicyclohexylcarbodiimide, where appropriate with the addition of N-hydroxysuccinimide, 1-hydroxybenzotriazole, 3-hydroxy-4-oxo-3,4-dihydro-1,2,3-benzotriazine or other racemization-reducing compounds.

7. The process as claimed in one of claims 1 - 3 for the preparation of a protected secretin derivative of the formula I

$$R-His(R^1)-Ser(Bu^t)-Asp(OBu^t)-Gly-Thr(Bu^t)-Phe-$$
$$Thr(Bu^t)-Ser(Bu^t)-Glu(OBu^t)-Leu-Ser(Bu^t)-Arg(HClO_4)-$$
$$Leu-Arg(HClO_4)-Asp(OBu^t)-Ser(Bu^t)-Ala-Arg(HClO_4)-Leu-$$
$$Gln-Arg(HClO_4)-Leu-Leu-Gln-Gly-Leu-Val-NH_2$$

$$(I)$$

in which
    a) R and $R^1$ denote the Boc radical or
    b) R denotes the Boc radical, and $R^1$ denotes hydrogen or
    c) R and $R^1$ denote the adamantyloxycarbonyl radical,
which comprises reaction of a peptide of the general formula IIa

H-X-Leu-Leu-Gln-Gly-Leu-Val-NH$_2$ (IIa)

in which X denotes

$$-Arg(HClO_4)-,$$
$$-Arg(HClO_4)-Leu-Gln-Arg(HClO_4)-,$$
$$-Arg(HClO_4)-Asp(OBu^t)-Ser(Bu^t)-Ala-Arg(HClO_4)-$$
$$\quad Leu-Gln-Arg(HClO_4)-$$

or

$$-Thr(Bu^t)-Ser(Bu^t)-Glu(OBu^t)-Leu-Ser(Bu^t)-Arg(HClO_4)-$$
$$Leu-Arg(HClO_4)-Asp(OBu^t)-Ser(Bu^t)-Ala-Arg(HClO_4)-Leu-$$
$$Gln-Arg(HClO_4)-$$

with the addition of 3-hydroxy-4-oxo-3,4-dihydro-1,2,3-benzotriazine with the appropriate peptides with free carboxyl group in polar solvents with dicyclohexylcarbodiimide and the necessary amounts of a tertiary base, and precipitation of the peptides, which have thus been synthesized, as perchlorates from water with the addition of appropriate amounts of perchloric acid and, where appropriate, of a perchlorate.

**8.** The process as claimed in claim 7, wherein the peptide of the general formula IIa is obtained by hydrogenation of benzyloxycarbonyl-containing fragments of the general formula II

Z-X-Leu-Leu-Gln-Gly-Leu-Val-NH$_2$ (II)

in which X denotes

$$-Arg(Z_2)-$$
$$-Arg(Z_2)-Leu-Gln-Arg(HClO_4)-$$
$$-Arg(Z_2)-Asp(OBu^t)-Ser(Bu^t)-Ala-Arg(HClO_4)-Leu-Gln-$$
$$\quad Arg(HClO_4)-$$

or

$$-\text{Thr}(\text{Bu}^{\text{t}})-\text{Ser}(\text{Bu}^{\text{t}})-\text{Glu}(\text{OBu}^{\text{t}})-\text{Leu}-\text{Ser}(\text{Bu}^{\text{t}})-\text{Leu}-$$
$$\text{Ser}(\text{Bu}^{\text{t}})-\text{Arg}(\text{HClO}_4)-\text{Leu}-\text{Arg}(\text{HClO}_4)-\text{Asp}(\text{OBu}^{\text{t}})-$$
$$\text{Ser}(\text{Bu}^{\text{t}})-\text{Ala}-\text{Arg}(\text{HClO}_4)-\text{Leu}-\text{Gln}-\text{Arg}(\text{HClO}_4)-$$

in dimethylacetamide with the addition of a palladium catalyst, the pH being maintained between 4 and 6 by addition of a solution containing perchloric acid, and is reacted further as claimed in claim 7.

9.  The process as claimed in one of claims 1 - 8, wherein pyridinium perchlorate is used for the preparation of the perchlorate of the arginine-containing fragment.

**Revendications**
**Revendications pour les Etats contractants suivants : BE, CH, DE, FR, GB, GR, IT, LI, LU, NL, SE**

1.  Procédé de préparation de peptides protégés contenant de l'arginine par conjugaison de fragments, procédé caractérisé en ce que dans chaque conjugaison de fragments on fait réagir au moins un fragment contenant de l'arginine, à l'état de perchlorate.

2.  Procédé selon la revendication 1, caractérisé en ce que l'on fait réagir à l'état de perchlorate une arginine éventuellement protégée.

3.  Procédé selon la revendication 1 ou 2, caractérisé en ce que l'on fait réagir à l'état de perchlorate un segment contenant de l'arginine.

4.  Procédé selon l'une des revendications 1 à 3, caractérisé en ce que au cours de la synthèse un fragment est lié à un corps solide par une liaison covalente.

5.  Procédé selon l'une des revendications 1 à 4, caractérisé en ce que l'on effectue la conjugaison en présence d'un carbodiimide.

6.  Procédé selon l'une des revendications 1 à 5, caractérisé en ce que l'on effectue la conjugaison en présence de N,N'-diisopropylcarbodiimide ou de N,N'-dicyclohexylcarbodiimide, et le cas échéant en ajoutant du N-hydroxysuccinimide, du 1-hydroxybenzotriazole, de la 3-hydroxy-4-oxo-3,4-dihydro-1,2,3-benzotriazine ou d'autres composés diminuant la racémisation.

7.  Procédé selon l'une des revendications 1 à 3 pour préparer un dérivé de sécrétine protégé de formule I ci-dessous :

$$\text{R}-\text{His}(\text{R}^1)-\text{Ser}(\text{Bu}^{\text{t}})-\text{Asp}(\text{OBu}^{\text{t}})-\text{Gly}-\text{Thr}(\text{Bu}^{\text{t}})-\text{Phe}-\text{Thr}(\text{Bu}^{\text{t}})-\text{Ser}(\text{Bu}^{\text{t}})-$$
$$\text{Glu}(\text{OBu}^{\text{t}})-\text{Leu}-\text{Ser}(\text{Bu}^{\text{t}})-\text{Arg}(\text{HClO}_4)-\text{Leu}-\text{Arg}(\text{HClO}_4)-\text{Asp}(\text{OBu}^{\text{t}})-$$
$$\text{Ser}(\text{Bu}^{\text{t}})-\text{Ala}-\text{Arg}(\text{HClO}_4)-\text{Leu}-\text{Gln}-\text{Arg}(\text{HClO}_4)-\text{Leu}-\text{Leu}-\text{Gln}-\text{Gly}-\text{Leu}-$$
$$\text{Val}-\text{NH}_2 \ (\text{I}),$$

formule dans laquelle
  a) R et $R^1$ sont le radical Boc ou bien
  b) R est le radical Boc et $R^1$ l'hydrogène, ou encore
  c) R et $R^1$ sont chacun le radical adamantyloxycarbonyle,
procédé caractérisé en ce que l'on fait réagir un peptide de formule IIa

H-X-Leu-Leu-Gln-Gly-Leu-Val-$NH_2$ (IIa)

X représentant :

```
-Arg(HClO₄)-,
-Arg(HClO₄)-Leu-Gln-Arg(HClO₄)-,
-Arg(HClO₄)-Asp(OButᵗ)-Ser(Buᵗ)-Ala-Arg(HClO₄)-Leu-Gln-
Arg(HClO₄)-
```

ou

```
-Thr(Buᵗ)-Ser(Buᵗ)-Glu(OButᵗ)-Leu-Ser(Buᵗ)-Arg(HClO₄)-Leu-
Arg(HClO₄)-Asp(OButᵗ)-Ser(Buᵗ)-Ala-Arg(HClO₄)-Leu-Gln-Arg(HClO₄),
```

en présence de 3-hydroxy-4-oxo-3,4-dihydro-1,2,3-benzotriazine (HOObt), avec les peptides correspondants à groupe carboxylique libre dans des solvants polaires, avec du dicyclohexylcarbodiimide et les proportions nécessaires d'une base tertiaire, puis on fait précipiter à l'état de perchlorates les peptides ainsi synthétisés, à partir d'eau, en ajoutant les proportions correspondantes d'acide perchlorique et le cas échéant d'un perchlorate.

**8.** Procédé selon la revendication 7, caractérisé en ce que l'on obtient le peptide de formule IIa en hydrogénant des fragments à groupes benzyloxycarbonyle de formule II

Z-X-Leu-Leu-Gln-Gly-Leu-Val-NH₂ (II)

dans laquelle X représente

```
-Arg(Z₂)-,
-Arg(Z₂)-Leu-Gln-Arg(HClO₄)-,
-Arg(Z₂)-Asp(OButᵗ)-Ser(Buᵗ)-Ala-Arg(HClO₄)-Leu-Gln-Arg(HClO₄)-
```

ou

```
-Thr(Buᵗ)-Ser(Buᵗ)-Glu(OButᵗ)-Leu-Ser(Buᵗ)-Arg(HClO₄)-Leu-Arg
(HClO₄)-Asp(OButᵗ)-Ser(Buᵗ)-Ala-Arg(HClO₄)-Leu-Gln-Arg(HClO₄)-,
```

dans du diméthylacétamide, en présence d'un catalyseur au palladium, tout en maintenant le pH du mélange entre 4 et 6 en ajoutant une solution d'acide perchlorique, puis on fait réagir ce peptide suivant la revendication 7.

**9.** Procédé selon l'une des revendications 1 à 8, caractérisé en ce que l'on forme avec du perchlorate de pyridinium le perchlorate du fragment contenant de l'arginine.

**10.** Peptide de formule I dans lequel R et R¹ ont les significations indiquées à la revendication 7.

**Revendications pour les Etats contractants suivants : AT, ES**

**1.** Procédé de préparation de peptides protégés contenant de l'arginine par conjugaison de fragments, procédé caractérisé en ce que dans chaque conjugaison de fragments on fait réagir au moins un fragment contenant de l'arginine, à l'état de perchlorate.

**2.** Procédé selon la revendication 1, caractérisé en ce que l'on fait réagir à l'état de perchlorate une arginine éventuellement protégée.

**3.** Procédé selon la revendication 1 ou 2, caractérisé en ce que l'on fait réagir à l'état de perchlorate un segment contenant de l'arginine.

**4.** Procédé selon l'une des revendications 1 à 3, caractérisé en ce que au cours de la synthèse un fragment est lié à un corps solide par une liaison covalente.

**5.** Procédé selon l'une des revendications 1 à 4, caractérisé en ce que l'on effectue la conjugaison en présence d'un carbodiimide.

**6.** Procédé selon l'une des revendications 1 à 5, caractérisé en ce que l'on effectue la conjugaison en présence de N,N'-diisopropylcarbodiimide ou de N,N'-dicyclohexylcarbodiimide, et le cas échéant en ajoutant du N-hydroxysuccinimide, du 1-hydroxybenzotriazole, de la 3-hydroxy-4-oxo-3,4-dihydro-1,2,3-benzotriazine ou d'autres composés diminuant la racémisation.

**7.** Procédé selon l'une des revendications 1 à 3 pour préparer un dérivé de sécrétine protégé de formule I ci-dessous :

$$R-His(R^1)-Ser(Bu^t)-Asp(OBu^t)-Gly-Thr(Bu^t)-Phe-Thr(Bu^t)-Ser(Bu^t)-$$
$$Glu(OBu^t)-Leu-Ser(Bu^t)-Arg(HClO_4)-Leu-Arg(HClO_4)-Asp(OBu^t)-$$
$$Ser(Bu^t)-Ala-Arg(HClO_4)-Leu-Gln-Arg(HClO_4)-Leu-Leu-Gln-Gly-Leu-$$
$$Val-NH_2 \quad (I),$$

formule dans laquelle
a) R et $R^1$ sont le radical Boc ou bien
b) R est le radical Boc et $R^1$ l'hydrogène, ou encore
c) R et $R^1$ sont chacun le radical adamantyloxycarbonyle,
procédé caractérisé en ce que l'on fait réagir un peptide de formule IIa

H-X-Leu-Leu-Gln-Gly-Leu-Val-NH$_2$ (IIa)

X représentant :

$$-Arg(HClO_4)-,$$
$$-Arg(HClO_4)-Leu-Gln-Arg(HClO_4)-,$$
$$-Arg(HClO_4)-Asp(OBu^t)-Ser(Bu^t)-Ala-Arg(HClO_4)-Leu-Gln-$$
$$Arg(HClO_4)-$$

ou

$$-Thr(Bu^t)-Ser(Bu^t)-Glu(OBu^t)-Leu-Ser(Bu^t)-Arg(HClO_4)-Leu-$$
$$Arg(HClO_4)-Asp(OBu^t)-Ser(Bu^t)-Ala-Arg(HClO_4)-Leu-Gln-Arg(HClO_4),$$

en présence de 3-hydroxy-4-oxo-3,4-dihydro-1,2,3-benzotriazine (HOObt), avec les peptides correspondants à groupe carboxylique libre dans des solvants polaires, avec du dicyclohexylcarbodiimide et les proportions nécessaires d'une base tertiaire, puis on fait précipiter à l'état de perchlorates les peptides ainsi synthétisés, à partir d'eau, en ajoutant les proportions correspondantes d'acide perchlorique et le cas échéant d'un perchlorate.

**8.** Procédé selon la revendication 7, caractérisé en ce que l'on obtient le peptide de formule IIa en

EP 0 224 844 B1

hydrogénant des fragments à groupes benzyloxycarbonyle de formule II

Z-X-Leu-Leu-Gln-Gly-Leu-Val-NH$_2$ (II)

dans laquelle X représente

```
-Arg(Z₂)-,
-Arg(Z₂)-Leu-Gln-Arg(HClO₄)-,
-Arg(Z₂)-Asp(OBuᵗ)-Ser(Buᵗ)-Ala-Arg(HClO₄)-Leu-Gln-Arg(HClO₄)-
```

ou

```
-Thr(Buᵗ)-Ser(Buᵗ)-Glu(OBuᵗ)-Leu-Ser(Buᵗ)-Arg(HClO₄)-Leu-Arg
(HClO₄)-Asp(OBuᵗ)-Ser(Buᵗ)-Ala-Arg(HClO₄)-Leu-Gln-Arg(HClO₄)-,
```

dans du diméthylacétamide, en présence d'un catalyseur au palladium, tout en maintenant le pH du mélange entre 4 et 6 en ajoutant une solution d'acide perchlorique, puis on fait réagir ce peptide suivant la revendication 7.

9. Procédé selon l'une des revendications 1 à 8, caractérisé en ce que l'on forme avec du perchlorate de pyridinium le perchlorate du fragment contenant de l'arginine.

21